# EUROPEAN PATENT SPECIFICATION

(11) **EP 3 793 372 B1**
(45) Date of publication and mention of the grant of the patent: **06.07.2022**
(21) Application number: 19730948.7
(22) Date of filing: 14.05.2019
(51) Int. Cl.: A23L 33/135, A61K 35/747, C12R 1/225

(54) **LACTOBACILLUS AMYLOVORUS SGL 14: PROBIOTIC ACTIVITY AND REDUCTION OF ENTERIC OXALATE**
LACTOBACILLUS AMYLOVORUS SGL 14: PROBIOTISCHE AKTIVITÄT UND REDUKTION VON ENTERALEN OXALATEN
LACTOBACILLUS AMYLOVORUS SGL 14: ACTIVITÉ PROBIOTIQUE ET RÉDUCTION DE L'OXALATE ENTÉRIQUE

(30) Priority: 14.05.2018 IT 201800005355
(43) Date of publication of application: 24.03.2021
(73) Proprietor: Nutraceutical Technology Pharma Biotech S.r.l., 64039 Penna Sant'Andrea (TE) (IT)
(72) Inventor: MARINI, Umberto, 64020 Castelnuovo Vomano (IT)
(74) Representative: Zaccaro, Elisabetta
(86) International application number: PCT/IB2019/053968
(87) International publication number: WO 2019/220328

(56) References cited:
- WO-A1-2013/050831
- US-A1- 2011 236 360
- LAURA MANNA ET AL: "Safety and efficacy of Phyllantin 14 in reducing urinary oxalate in a mouse model of dietary hyperoxaluria", VITAFOODS EUROPE EDUCATION PROGRAMME 2019 GENEVA, 7 May 2019 (2019-05-07), page 1, XP055605298,

### SEQUENCE LISTING

<110> SINTAL DIETETICS S.R.L.
<120> Lactobacillus amylovorus SGL 14: probiotic activity and reduction of
   enteric oxalate
<130> P020513WO-01
<160> 6
<170> BiSSAP 1.3.6
<210> 1
   <211> 20
   <212> DNA
   <213> Artificial Sequence
<220>
   <223> 16S-27 For Primer
<400> 1
   agagtttgat cctggctcag 20
<210> 2
   <211> 19
   <212> DNA
   <213> Artificial Sequence
<220>
   <223> 16-1552 Rev Primer
<400> 2
   aaggaggtgw tcarccgca 19
<210> 3
   <211> 22
   <212> DNA
   <213> Artificial Sequence
<220>
   <223> oxc-L Primer
<400> 3
   cttgaaatgc aagatgaaag ca 22
<210> 4
   <211> 22
   <212> DNA
   <213> Artificial Sequence
<220>
   <223> oxc-R Primer
<400> 4
   cttcagtcat tatttattct cc 22
<210> 5
   <211> 23
   <212> DNA
   <213> Artificial Sequence
<220>
   <223> frc-L Primer
<400> 5
   ggagaataaa taatgactga aga 23
<210> 6
   <211> 22
   <212> DNA
   <213> Artificial Sequence
<220>
   <223> frc-R Primer
<400> 6
   cggtaaaaat taattattca cc 22

## Description

### FIELD OF THE INVENTION

The present invention concerns a bacterial strain with probiotic activity having the ability to reduce the amounts of enteric oxalate. Compositions comprising such bacterial strain, and food and medicament use thereof, are also described.

### STATE OF THE ART

Probiotics are living microorganisms that can bring beneficial properties to the body, if they are included into the diet in adequate amounts. For an organism to be considered "probiotic", there must be scientific studies that ensure on the one hand its safe use in humans and, on the other hand, the presence of properties such as resistance to the aggression of gastric, pancreatic and bile juices, and the ability to adhere firmly to the intestinal mucosa by colonizing it.

Lactic Acid Bacteria (LAB), for the most part represented by lactobacilli and bifidobacteria, are the most common types of probiotic microorganisms.

Such microorganisms can, directly or indirectly, carry out their effect at the level of the host organism by modulating the endogenous ecosystem or the immune response. Some probiotics, on the other hand, can act by directly strengthen the intestinal barrier, preventing permeability, and the subsequent loss of macromolecules, which are observed in intestinal infections and food intolerances, or by exerting a trophic action on the colon mucosa, or by protecting the mucus coating the intestinal wall. Yet other species indirectly intervene on the intestinal barrier by stimulating the intestinal immune system, or Gut Associated Lymphoid Tissue (GALT), which constitutes an immune defense barrier for the body. Specifically, probiotics are co-adjuvants of the endogenous bacterial flora, ensuring the development of IgA producing cells and intestinal epithelial lymphocytes, as well as modulating the production of IgE and interleukins. Basic research also showed that probiotics can act by inhibiting the synthesis of some inflammatory mediators. Recently, studies on probiotics have focused on their beneficial properties directed towards well-defined therapeutic conditions, such as hypocholesterolizing activity, anti-stress activity, modulating activity in dermatitis, etc.

The application that has already been partly studied and clarified is the role of lactobacilli in reducing enteric oxalate. In fact, at the intestinal level, they are able to consume the oxalate salts ingested with the diet and reduce the absorption thereof, by acting preventively on the onset of renal diseases such as hyperoxaluria, lithiasis, and calculosis.

Accumulation of oxalate in humans occurs on two levels: endogenous metabolic production by the liver system and absorption of dietary oxalate in the gastrointestinal tract. An imbalance between production/absorption and excretion can lead to hyperoxaluria, which in turn is the primary risk factor for the appearance of kidney stones. To date, there are no dedicated pharmacological treatments: we try to balance the diet in order to minimize the intake of oxalate to prevent its occurrence of kidney stones and in their presence, we resort to the surgical practices for removing stones. Also in this case, the problem reappears with frequent recurrences in up to 50% of the treated patients.

In recent years, the crucial role of intestinal microbiota has emerged as a factor that influences the intestinal homeostasis of oxalate salts. The absence of oxalatedegrading microorganisms is always associated with the pathology, and manipulation of microflora composition has been proposed as a therapeutic approach to prevention and maintenance.

Also plant extracts have been studied in order to exploit their therapeutic properties in this field, and beneficial effects of *Phyllanto niruri* have been demonstrated on the formation process and morphology of kidney stones.

The object of the present invention is therefore to provide a bacterial strain having probiotic activity with enteric oxalate-lowering activity, thus allowing to avoid the need for surgical intervention and and excessive use of drugs.

### SUMMARY OF THE INVENTION

The invention therefore concerns a bacterial strain belonging to the *Lactobacillus amylovorus* species, wherein said strain is *L. amylovorus* SGL 14 deposited with accession number DSM 27014 on 18/03/2013 at the International Depositary Authority Leibniz-lnstitut DSMZ - Deutsche Sammlung von Mikroorganismen und Zellkulturen GmbH.

The *L. amylovorus* SGL 14 DSM 27014 strain was deposited in the name of MD'E srl, via Nazionale 339, Castelnuovo Vomano (TE) 64020 Italy, which together with Sintal Dietetics is part of the same network of companies.

In a further aspect, the invention concerns a composition comprising the bacterial strain *L. amylovorus* SGL 14, optionally added with pharmacologically acceptable excipients.

The invention further concerns a composition comprising the bacterial strain L. *amylovorus* SGL 14, and a plant extract of *Phyllanto niruri,* optionally added with pharmacologically acceptable excipients.

In a further aspect, the invention relates to a composition comprising the bacterial strain *L. amylovorus* SGL 14, and the bacterial strain *L. gasseri* SGL 18 deposited with accession number DSM 32802 on April 20, 2018 at the International Depositary Authority Leibniz-lnstitut DSMZ - Deutsche Sammlung von Mikroorganismen und Zellkulturen GmbH optionally added with pharmacologically acceptable excipients. In a further aspect, the invention concerns the composition comprising the bacterial strain *L. amylovorus* SGL 14, for use as a medicament.

In particular, said medicament is for use in the treatment and prevention of calculi selected from the group consisting in: kidney stones, gallstones and gallbladder stones, and for use in the treatment and prevention of hyperoxalurias and lithiasis

The present invention further concerns the composition comprising the bacterial strain *L. amylovorus* SGL 14, the composition comprising the bacterial strain *L*. *amylovorus* SGL 14, a plant extract of *Phyllanto niruri* and the composition comprising the bacterial strain *L. amylovorus* SGL 14 and the bacterial strain *L*. *gasseri* SGL 18 deposited with accession number DSM 32802 on April 20, 2018 for food use.

The disclosure also relates to the bacterial strain *L. gasseri* SGL 18 deposited with accession number DSM 32802 on April 20, 2018 at the International Depositary Authority Leibniz-lnstitut DSMZ - Deutsche Sammlung von Mikroorganismen und Zellkulturen GmbH. The strain *L. gasseri* SGL 18 DSM 32802, was deposited in the name of MD'E srl, via Nazionale 339, Castelnuovo Vomano (TE) 64020 Italy, which together with Sintal Dietetics is part of the same network of companies.

### DESCRIPTION OF THE FIGURES

The invention will now be described in detail, and with reference to the attached Figures wherein:
Figure 1 depicts the graph of the results obtained from the oxalate concentration measurements in the culture media shown in Table 6 and described in Example 5;
Figures 2A and 2B depict the graphs of the results showing both the oxalate mM values consumed in the 27 days of monitoring, and the derived percentage values described in Example 5.
Figure 3 depicts the graph relating to the decrease in urinary oxalate in animal models treated with a supplement prototype comprised of the association of *L*.
*amylovorus* SGL 14 and dry extract of *Phyllanto niruri.* The graph shows 4 curves wherein: curve 1 corresponds to the negative control group fed with normal diet,
curve 2 to the toxicity test group fed with normal diet and high-dose supplement prototype, curve 3 to the positive control group fed with high-ox diet, and curve 4 to the efficacy test group fed with high-ox diet, with the addition of the high-dose supplement prototype.

### DETAILED DESCRIPTION OF THE INVENTION

The invention therefore concerns a bacterial strain belonging to the *Lactobacillus amylovorus* species, wherein said strain is *L. amylovorus* SGL 14 deposited with accession number DSM 27014 on 18/03/2013 at the International Depositary Authority Leibniz-lnstitut DSMZ - Deutsche Sammlung von Mikroorganismen und Zellkulturen GmbH.

Advantageously, the inventors have shown that the strain *L. amylovorus* SGL 14 has probiotic activity and brings benefit to the organism. In particular, this strain allows the reduction of enteric oxalate, favoring the intestinal homeostasis of oxalate salts, preventing the accumulation and formation of kidney stones.

In the present invention, when using the definition:
- "probiotic" or "probiotic activity" means a living, non-pathogenic microorganism present in food or added to it which, when administered in adequate amounts, brings a benefit to the health of the host. Lactic acid bacteria (LAB, Lactic Acid Bacteria), mostly represented by lactobacilli and bifidobacteria are the most common types of probiotic microorganisms.

In a further aspect, the invention concerns a composition comprising the bacterial strain *L. amylovorus* SGL 14, optionally added with pharmacologically acceptable excipients.

The bacterial strain *L. amylovorus* SGL 14 is advantageously prepared in the form of a composition. Suitable compositions may be in the form of powders, capsules, tablets, granulates, drops and syrups, chewing gum. The ranges of use of the ferment are from 1 billion/dose to 20 billion/dose, preferably such a range is from 200 billion/dose to 20 billion/dose, more preferably from 50 billion/dose to 40 billion/dose.

The invention further concerns a composition comprising the bacterial strain *L*. *amylovorus* SGL 14, and a plant extract of *Phyllanto niruri* optionally added with pharmacologically acceptable excipients. The possible compositions may simple be premixed powders, finished pharmaceutical forms: capsules, tablets, sachets, drops and syrups, chewing gum. The ranges of use of the ferment are from 1 billion/dose to 20 billion/dose for the ferment, and from 0.5 g/dose to 10 g/dose for the extract of *Phyllanto niruri.*

In a further aspect the invention relates to a composition comprising the bacterial strain *L. amylovorus* SGL 14 and the bacterial strain *L. gasseri* SGL 18 deposited with accession number DSM 32802 on April 20, 2018 at the International Depositary Authority Leibniz-lnstitut DSMZ - Deutsche Sammlung von Mikroorganismen und Zellkulturen GmbH optionally added with pharmacologically acceptable excipients. The present invention further concerns the composition comprising the bacterial strain *L. amylovorus* SGL 14, the composition comprising the bacterial strain *L*. *amylovorus* SGL 14, a plant extract of *Phyllanto niruri,* and the composition comprising the bacterial strain *L. amylovorus* SGL 14 and the bacterial strain *L*. *gasseri* SGL 18 deposited with accession number DSM 32802 on April 20, 2018 for food use.

The present invention concerns a beverage containing the composition comprising the bacterial strain *L. amylovorus* SGL 14, the composition comprising the bacterial strain *L. amylovorus* SGL 14, a plant extract of *Phyllanto niruri,* and the composition comprising the bacterial strain *L. amylovorus* SGL 14 and the bacterial strain *L*. *gasseri* SGL 18 deposited with accession number DSM 32802. Preferably said beverage is a fruit juice, coffee, tea, and a soft drink.

Advantageously the composition comprising:
- the bacterial strain *L. amylovorus* SGL 14;
- the bacterial strain *L. amylovorus* SGL 14 and a plant extract of *Phyllanto niruri*;
- the bacterial strain *L. amylovorus* SGL 14 and the bacterial strain *L. gasseri* SGL 18; and
- the bacterial strain *L. amylovorus* SGL 14, a plant extract of *Phyllanto niruri* and the bacterial strain *L. gasseri* SGL 18, has probiotic activity.

The compositions according to the invention may be in a solid or liquid form. In the compositions according to the present invention, the dosages are in a range from 1 billion/dose to 20 billion/dose for ferments, preferably this range is from 200 billion/dose to 20 billion/dose, more preferably from 50 billion/dose to 40 billion/dose, SGL 14 and SGL 18, and in a range from 0.5 g/dose to 10 g/dose for the extract of *Phyllanto niruri.*

Suitable compositions may be in the form of powders, capsules, tablets, granulates, drops and syrups, chewing gums. In a further aspect, the invention concerns the composition comprising the bacterial strain *L. amylovorus* SGL 14, for use as a medicament.

In particular, said medicament is for the use in the treatment and prevention of calculi selected from the group consisting of: kidney stones, gallstones and gallbladder stones, and for use in the treatment of hyperoxalurias, clinical conditions in which there is an increased urinary excretion of calcium oxalate that can lead to formation of kidney stones, and lithiasis.

The compositions according to the present invention have surprising activity in the treatment of calculi and lithiasis, which very often require surgical interventions and/or pharmacological treatments. By using the bacterial strain according to the present invention, these diseases may be prevented and treated in a more natural way.

In a further aspect, the disclosure relates to the bacterial strain *L. gasseri* SGL 18 deposited with accession number DSM 32802 on April 20, 2018 at the International Depositary Authority Leibniz-lnstitut DSMZ - Deutsche Sammlung von Mikroorganismen und Zellkulturen GmbH.

Examples of embodiments of the present invention are given below by way of illustration.

### EXAMPLES

### Example 1

### Strain Isolation

The strain was isolated from feces starting from feces of pig in healthy conditions in MRS under anaerobic conditions at 37°C for 48 hours. *L. amylovorus* SGL 14 colonies are creamy-white in color, not too small with a starry appearance. The morphology of the microorganism under the microscope may be described as rodshaped microorganisms with medium-length, rather thick, regular rods.

### Materials and Methods

The *L. amylovorus* SGL 14 strain was isolated starting from feces of animals in healthy conditions.

Initially, a suspension of fresh feces in a complex medium, 50% de Man, Rogosa and Sharpe (MRS) broth (Oxoid, Basingstoke, UK) added with 0.05% L-cysteine as a reducing agent, was prepared. After incubating the samples at 37°C for 30 minutes, the isolation in MRS was carried out.

### Example 2

### Taxonomic identification: Molecular Characterization

Lactobacilli strains selected by isolation with MRS medium were unambiguously taxonomically classified by the study of the entire 16S gene; a 1500 bp long region was amplified using generic primers for eubacteria, sequenced, and then aligned using BLAST with public databases. The results enabled a certain classification of the strain that was ascribed to the *Lactobacillus amylovorus* species. The Maldi Tof results shown below provided additional support to the strain identification.

| | | | | | |
|---|---|---|---|---|---|
| **C3 (++B)** | **20** | **Lactobacillus amylovorus** | **2.137** | **Lactobacillus amylovorus** | **2.118** |

The *L. amylovorus* SGL 14 strain was deposited at the European International Collection DSMZ, an organization recognized with the IDA (International Depositary Authority) status, with a "patent deposit" having the following data: *L. amylovorus* SGL 14 DSM 27014, on 18/03/2013.

### Materials and Methods

### Taxonomic identification

The isolated strain was initially identified by growth in MRS medium, as described in Example 1, and direct observation of the morphology of the plate-grown colonies and cell morphology following observation by light microscopy.

The certain attribution of the genus and the species was carried out, instead, by techniques of analysis of 16S ribosomal DNA and biochemical characterization.

### Analysis of sequence 16S

The microorganism genome was extracted using commercial kits (Macherey Nagel, Düren, Germany) following manufacturer's indications. DNA was used in the concentration of 50-100 ng for the amplification reaction of 16S gene. Amplification reactions were performed using Biometra T-professional thermocycler (Biometra, Göttingen, Germany). The amplification conditions used were those that gave the best yields of amplified gene. All reactions were carried out in a final volume of 25 µL.

The components used in all amplification reactions are:
- DyNAzyme (Finnzymes, Espoo, Finland) as thermostable DNA polymerase (1 U per reaction) and its reaction buffer 10x Optimized DyNAzyme buffer (100 mM Tris-HCI, 500 mM KCI, 15 mM MgCl₂, 0.1% Triton X-100, pH 8.8 at 25°C);
- mixture of triphosphate nucleotides (8 mM, Euroclone, Siziano, Italy).
- primer set (100 µM, Eurofins MWG Operon, Ebersberg, Germany);
- genomic DNA;
- sterile water.

The following are the primers used, the thermocycle and the expected amplicon. Primer: 16S-27 For 5'-AGAGTTTGATCCTGGCTCAG-3' SEQ ID NO:1 16-1552 Rev 5'-AAGGAGGTGWTCARCCGCA-3' SEQ ID NO:2

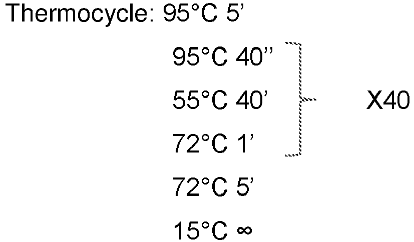

Amplicon: 1552 bp

The fragment of about 1550 bp was sequenced (MWG Biotech, Ebersberg, Germany) and was aligned with Blast database (NCBI, nucleotide blast) to obtain the correct identification of the species.

### Strain profile in MALDI-TOF MS

To obtain a identifier Maldi Tof profile, the sample was prepared as follows:
a bacterial culture of *L. amylovorus* SGL 14 grown overnight was collected in order to have a sufficient number of cells for detection (5 billion). The cell pellet was washed with saline solution to remove residues of fermented medium and resuspended in 300 µL of distilled water and 900 µL of pure ethanol. The suspension was well homogenized. The sample was then centrifuged at 13,000 rpm for 2 min and dried in the air after removing the supernatant. The sample was stored at -20°C pending analysis.

At the time of analysis, the sample was resuspended with 50 µL of 70% formic acid. After resuspension, additional 50 µL of acetonitrile were added and the whole was vigorously stirred. The sample was then centrifuged for 2 min in a table-top centrifuge at 13,000 rpm. 1 µL of supernatant was placed onto the center of a spot of a disposable target plate and air-dried. The addition of 1µL of matrix made of organic materials (α-cyano-4-hydroxycinnamic acid) started the Maldi Tof analysis. Maldi Tof MS was performed with a MicroFlex LT system using the conditions included in the user manual. The ions generated by a nitrogen laser, at a 337 nm wavelength, were linearly captured in a mass range of 2 to 20 KDa. The instrument is connected to the Biotyper 2.0 software (Bruker Daltonics) capable of acquiring incoming data, elaborate them in mass spectra and compare them with reference spectra present in the internal database (3,740 spectra of 319 genres and 1,946 different species). For each 24-sample plate, a standard test was included to calibrate and validate the analysis.

The degree of correspondence with the typical spectrum for each microorganism present in the database determines the attribution of a so-called "score value" expressing the degree of certainty for the proposed identification of the species under examination. Scores with a value greater than 2,000 are considered reliable for identification at the species level, values between 1,700 and 1,900 give a certain identification at the genus level.

The strain was analyzed in triple on 3 different bacterial cultures.

### Example 3:

### Taxonomic Identification: Biochemical Characterization

The *L. amylovorus* SGL 14 strain was also analyzed for the biochemical profile by Gram staining, oxidase test, catalase test and API 50CH system.

The results obtained confirmed that: the strain is gram positive; catalase and oxidase negative, and it ferments sugars according to the profile below.

**Table 1:**

| | |
|---|---|
| Arbutin | NEG |
| Amygdalin | NEG |
| N-acetylglucosamine | POS |
| Methyl-aD-g lucopyranose | NEG |
| Methyl-aD-mannopyranose | NEG |
| D-sorbitol | NEG |
| D-mannitol | NEG |
| Inositol | NEG |
| Dulcitol | NEG |
| L-rhamnose | NEG |
| L-sorbose | NEG |
| D-mannose | POS |
| D-fructose | POS |
| D-glucose | POS |
| D-galactose | POS |
| Methyl-βD- xylopyranose | NEG |
| Adonitol | NEG |
| L-xylose | NEG |
| D-xylose | NEG |
| D-ribose | NEG |
| L-arabinose | NEG |
| D-arabinose | NEG |
| Erythritol | NEG |
| Glycerol | NEG |
| Control | NEG |
| Arbutin | NEG |
| Amygdalin | NEG |
| N-acetylglucosamine | POS |
| Methyl-aD-g lucopyranose | NEG |
| Methyl-aD-mannopyranose | NEG |
| D-sorbitol | NEG |
| D-mannitol | NEG |
| Inositol | NEG |
| Dulcitol | NEG |
| L-rhamnose | NEG |
| L-sorbose | NEG |
| D-mannose | POS |
| D-fructose | POS |
| D-glucose | POS |
| D-galactose | POS |
| Methyl-βD-xylopyranose | NEG |
| Adonitol | NEG |
| L-xylose | NEG |
| D-xylose | NEG |
| D-ribose | NEG |
| L-arabinose | NEG |
| D-arabinose | NEG |
| Erythritol | NEG |
| Glycerol | NEG |
| Control | NEG |

### Materials and Methods

### Gram Staining

Gram staining is a type of differential staining that allows to classify bacteria in Gram-positive and Gram-negative.

This identification test, which was carried out using the Gram Color Kit (Liofilchem, Teramo, Italia), is divided into 4 steps, spaced by washings with distilled water, each of them involving treatment with a particular reagent that is allowed to act for about 1 min on a loop of cells from a culture on solid medium, dissolved with a drop of sterile water on a slide and heat fixed with a Bunsen burner.

The reagents used, in order of application, are:
- crystal violet,
- Lugol's solution,
- decolorizing solution (ethyl alcohol),
- safranin.

At the end, Gram-positive bacteria appear purple in color, while Gram-negative bacteria are red or pink.

### Sugar Fermentation Profile (API)

For the biochemical characterization of *L. salivarius* SGL03, the API 50 CH gallery system was used with API 50 CHL (BioMérieux, Milano, Italy) inoculum medium. The gallery consists of 50 microtubes containing dehydrated substrates for detection of the enzymatic activity and fermentation of sugars. The bacterial suspension, having a turbidity of 2 McFarland, was distributed in the microtubes, and anaerobiosis was obtained by covering it with sterile paraffin oil. Substrate metabolism by the bacterium during the incubation period at 37°C is highlighted by a color change in the medium, following acidification of the medium. Reading of the reaction results was performed on the basis of a reading table where the results were recorded, and the identification was performed using a computer software (www.apiweb.biomeneux.com).

### Catalase Assay

This assay is used to verify the presence of catalase enzyme, able to detoxify hydrogen peroxide, in a bacterial culture.

A loop of cells from a culture on solid medium were dissolved in a drop of 30% hydrogen peroxide; immediate appearance of effervescence is indicative of catalase presence.

Effervescence is due to the oxygen that develops in the reaction:

2 H₂O₂ → 2 H₂O + O₂

### Oxidase Assay

The oxidase assay is an enzymatic test used to detect the presence of cytochrome oxidase enzyme in bacteria; it is therefore positive for aerobic and facultative anaerobic bacteria.). Oxidase Test Discs (Liofilchem, Teramo, italia), *i.e.* 3 mm diameter paper discs impregnated with tetramethyl-p-phenylenediamine dihydrochloride (Kovacs reagent), were used. The disc was moistened with 2 drops of distilled water and rubbed with a loop of a pure colony.

The development of a blue color within 30 seconds demonstrates oxidation of the substrate contained in the disk, the microorganism is therefore oxidase-positive; conversely, if no color development occurs, the microorganism will be oxidasenegative.

### Example 4

### Tolerance to gastro-intestinal environment

The microorganism was tested in order to ensure its gastro-intestinal transit and guarantee it could survive and reach, remaining fully viable, the intestinal section where it has to perform its physiological activity.

This strain was therefore tested under the following environmental conditions:
1. resistance to pepsin in acid environment
2. resistance to pancreatin in basic environment
3. resistance to high bile salts concentration
4. resistance to acid environment
5. resistance to high salts concentrations

*L. amylovorus* SGL 14 showed a good tolerance to gastro-intestinal environment. In particular, the results showed a good survival rate after 1h, and also after 4h incubation, in the presence of pancreatin at pH 8. Only a slight decrease is observed at 4 hours, both in the test and in the control, indicating that the effect is due to pH. Similar results were obtained after 1 hour and 30 minutes and after 2 hours and 30 minutes of incubation, in the presence of pepsin at pH 3. The results obtained are shown in Table 2 and 3 below.

**Table 2:**

| ***Resistance to Pepsin and Pancreatin*** | | | |
|---|---|---|---|
| **PEPSIN pH3** | **LOG10 CFU/mL *L amylovorus* SGL 14.** | | |
| | **Test** | **t0** | 7.98±0.2 |
| | | **t90'** | 7.85±0.18 |
| | | **t120'** | 7.74±0.23 |
| | | **% Survival @90'** | 98.35±5 |
| | | **% Survival @120'** | 96.9±7 |
| | **Control** | **t0** | 8.09±0.11 |
| | | **t90'** | 7.86±0.09 |
| | | **t120'** | 7.17±11 |
| | | **% Survival @90'** | 97.16±5 |
| | | **% Survival @120'** | 94.03±8 |
| **PANCREATIN pH8** | **Test** | **t0** | 8.12±0.03 |
| | | **t60'** | 8.01±0.12 |
| | | **t240'** | 5.78±0.07 |
| | | **% Survival @60'** | 98.70±9 |
| | | **% Survival @240'** | 71.20±12 |
| | **Control** | **t0** | 7.95±0.018 |
| | | **t60'** | 8.02±0.02 |
| | | **t240'** | 7.35±0.21 |
| | | **% Survival @60'** | 100.82±5 |
| | | **% Survival @240'** | 92.45±7 |

**Table 3:**

| ***Resistance to Bile Salts*** | | | |
|---|---|---|---|
| | **LOG10 (CFU/mL)** | | ***L. amylovorus* SGL 14** |
| **Bile Salts** | **t0** | **t 24h** | **Growth** |
| **0%** | 7.64±0.22 | 7.74±0.19 | Normal |
| **0.5%** | 7.49±0.22 | 6.59±0.22 | Slight decrease |
| **1%** | 7.45±0.25 | 5.63±0.21 | Slight decrease |
| **2%** | 7.43±0.27 | 6.65±0.26 | Slight decrease |
| **3%** | 7.46±0.28 | 7.27±0.25 | Normal |

The microorganism is slightly affected by the presence of bile salts at concentrations higher than 0.5% (about 2.5 times higher than the physiological ones), showing a good survival also in the presence of 3% bile salts. This makes it suitable for probiotic use.

**Table 4:**

| | **LOG10 (CFU/mL)** | | **SGL14** | | **LOG10 (CFU/mL)** | | **SGL 14** |
|---|---|---|---|---|---|---|---|
| **pH** | **t0** | **t 24h** | **Growth** | **NaCl** | **t 0** | **t 24h** | **Growth** |
| **1.5** | 7.27±0.02 | <4 | No growth | **0%** | 7.65±0.3 | 5.78±0.2 | Control |
| **2** | 7.84±0.05 | <4 | No growth | **2%** | 7.82±0.2 | 7.49±0.2 | Normal |
| **3** | 7.87±0.1 | 6.18±0.02 | Normal | **6%** | 7.67±0.15 | <3 | No growth |
| **6.3** | 7.90±0.04 | 5.48±0.04 | Control | **10%** | 7.63±0.2 | <3 | No growth |

*L. amylovorus* SGL 14 shows a low tolerance to acid pH; at a pH below 3 its survival is compromised. It is equally affected by the presence of NaCl in the culture medium, its survival is ensured up to 2% NaCl while at higher concentrations the microorganism ceases to grow.

### Materials and Methods

### Characterization of probiotic characteristics of L. amylovorus SGL 14

### -Resistance Test to Gastro-intestinal tract Environment

In order to evaluate the tolerance to gastro-intestinal juices, viability of the isolated strain over time was determined by plate count, after it had been contacted with:
- synthetic pepsin;
- synthetic pancreatin;
- various concentrations of bile salts;
- various concentrations of NaCl;
- acid pH growth medium.

### - Pepsin Resistance Test

A pepsin solution consisting of 3 g/L of pepsin, derived from pig gastric mucosa (Sigma-Aldrich, St. Louis, MO), was obtained by dissolving powdered pepsin in sterile 0.5% NaCl (w/v) saline solution, and the pH was adjusted to 3. Starting from cultures grown overnight in 10 mL of MRS broth medium supplemented with 0.05% of L-cysteine, at 37°C for about 18 hours, in the presence of O₂, about 10⁹ cells/mL were harvested by centrifugation at 4,000 rpm for 10 min and, with the same centrifugation cycle, they were washed with a 0.09% NaCl (w/v) sterile saline solution.

The cells were then washed a second time with a 0.09% NaCl (w/v) sterile saline solution and, to simulate passage through the gastric tract, cells were resuspended in 10 mL of pepsin solution at pH 3. The suspension thus prepared was incubated in anaerobiosis, at 37°C for 2 hours, measuring the number of CFU/mL at 0, 90 and 120 minutes by plate count.

To monitor growth in the absence of pepsin, the strain under examination was inoculated into two 0.5% (w/v) sterile saline solutions, one at pH 3 and incubated under the same conditions, to be used as a control in resistance calculation.

The resistance of the treated strain was expressed as a percentage of survival over the control.

### - Pancreatin Resistance Test

A pancreatin solution, consisting of 1 g/L pancreatin from pig pancreas (Sigma-Aldrich), was obtained by dissolving the powder in 0.5% NaCl (w/v) sterile saline solution, and the pH was adjusted to 8. Starting from cultures grown overnight in 10 mL of MRS broth medium supplemented with 0.05% of L-cysteine, at 37°C for about 18 hours, in the presence of O₂, about 10⁹ cells/mL were harvested by centrifugation at 4,000 rpm for 10 min and, with the same centrifugation cycle, they were washed with a 0.09% NaCl (w/v) sterile saline solution.

Subsequently, the cells were neutralized by washing with a sterile phosphate buffer (PBS - 8 g/L NaCl, 0.2 g/L KCI, 1.44 g/L Na₂HPO₄, 0.24 g/L KH₂PO₄) at pH 7 and, to simulate passage through the intestine, they were resuspended in 10 mL of pancreatin solution at pH 8. The suspension was incubated in anaerobiosis, at 37°C for 4 hours, and the number of CFU/mL at 0, 60 and 240 minutes was estimated by plate count.

To monitor growth in the absence of pancreatin, the strain under evaluation was inoculated into two 0.5% (w/v) sterile saline solutions, at pH 8 respectively, and incubated under the same conditions, to be used as a control in resistance calculation.

The resistance of the treated strain was expressed as a percentage of survival over control.

### - Resistance Test to Bile Salts, pH and NaCl

The analysis was carried out on bacterial cultures grown overnight. Approximately 10⁹ cells/mL were harvested by centrifugation at 4,000 rpm for 10 minutes. The cells were then washed with 2 mL of sterile physiological solution (NaCl 9 g/L) and, following centrifugation at 4,000 rpm for 10 minutes, they were resuspended in MRS broth containing:
- 0%, 0.5%, 1%, 2%, 3% bile salts (w/v);
- 0%, 2%, 6%, 10% NaCI (w/v);
- pH 6.3, 3, 2, 1.5 adjusted with 4 N HCI.

The solutions were incubated in anaerobiosis, at 37°C for 24h, for each inoculum the optical density at 600 nm was recorded at 0h, 2h and 24h, while the estimate of the CFU/mL number was performed at 0h and 24h, by plate count.

### Example 5

### Antibiotic Resistance Profile

The table reported below shows the resistance profile to antibiotics tested against the *L. amylovorus* SGL 14 strain, by using the MIC Test Strip.

**Table 5:**

| **ANTIBIOTIC** | **FAMILY** | **MECHANISM OF ACTION** | **MIC (µg/mL)** |
|---|---|---|---|
| Amoxicillin | β-lactam | Cell wall synthesis inhibitor | 0.19 |
| Ampicillin | β-lactam | Cell wall synthesis inhibitor | 0.38 |
| Clindamycin | Lincosamide | Protein synthesis inhibitor | >256 |
| Chloramphenicol | Macrolide | Protein synthesis inhibitor | 4 |
| Ciprofloxacina | Quinolone | DNA synthesis inhibitor | >32 |
| Erythromycin | Macrolide | Protein synthesis inhibitor | 48 |
| Gentamycin | Aminoglycoside | Protein synthesis inhibitor | 32 |
| Kanamycin | Aminoglycoside | Protein synthesis inhibitor | >256 |
| Rifampicin | Rifampicin | Protein synthesis inhibitor | 0.19 |
| Streptomycin | Aminoglycoside | Protein synthesis inhibitor | >1024 |
| Sulfamethoxazole | Sulfamide | DNA synthesis inhibitor | >1024 |
| Tetracycline | Tetracycline | Protein synthesis inhibitor | 32 |
| Vancomycin | Glycopeptide | Cell wall synthesis inhibitor | 0.5 |

### Materials and Methods

### Antibiotic Resistance Profile

Sensitivity to antimicrobial agents of the *L. salivarius* SGL03 strain was tested using MIC Test Strip (Liofilchem), a quantitative method for determination of the minimum inhibitory concentration (MIC) of a single antimicrobial agent against microorganisms. The kit consists of paper strips impregnated with a predetermined gradient of antibiotic concentrations, consisting of 15 dilutions in the range of dilutions used in conventional methods for MIC determination.

The cultures in stationary phase were diluted in MRD buffer (Liofilchem) in order to have a turbidity equal to McFarland 3 standard. 100 µL of this solution were spatulated on the surface of MRS agar medium supplemented with 0.05% of L-cysteine in plate, in order to produce a homogeneous growth. After allowing the agar surface to dry completely, the strip was placed in the center of the plate with the MIC values facing upward. The plates thus prepared were incubated, with the lid facing upward, in aerobiosis, at 37°C for 48 hours.

When the kit strip is applied to the inoculated surface of plated agarized medium, the pre-defined gradient is released by the strip to the medium, therefore, after the incubation, an elliptical, symmetrical, and centered along the strip inhibition area can be observed. The MIC value, expressed in µg/mL, was read at the intersection point between the edge of the inhibition ellipse and the paper strip.

### Example 6

### Adhesion to Intestinal Cells Monolayer

The adhesion activity of the *L. amylovorus* SGL 14 strain to the human enterocyte line HT29 was evaluated by counting the number of microorganisms adhered to HT29 cells by Real time PCR. The result obtained was 2.39E+3/100 HT29 cells; *L*. *amylovorus* SGL 14 may be therefore considered a medium-adhesive strain (adhesion reference value *Salmonella* 6.77E+5/100 HT29 cells and 157 of E. *coli* B44/100 HT29 cells).

### Materials and Methods

### Adhesion to Intestinal Cells Monolayer

To assess the adhesion activity of the *L. amylovorus* SGL 14 strain to the human enterocyte line HT29, 1 × 10⁸ bacterial cells were added to an epithelial monolayer of HT29 cells (1.23 × 10⁶ cells) and incubated for 1.5 h. At the end of the incubation, the cell monolayer was washed 4 times with PBS, and the adherent bacterial cells were quantified by Real Time PCR using the genus-specific primer.

The adherent enteropathogens *Salmonella Typhimurium* and *E.coli* ETEC H10407 were used as positive controls, while the non-adherent *E*. *coli* B44 strain was used as negative control.

### Example 7

### Assessment of oxalate degradation activity in probiotic strains in vitro

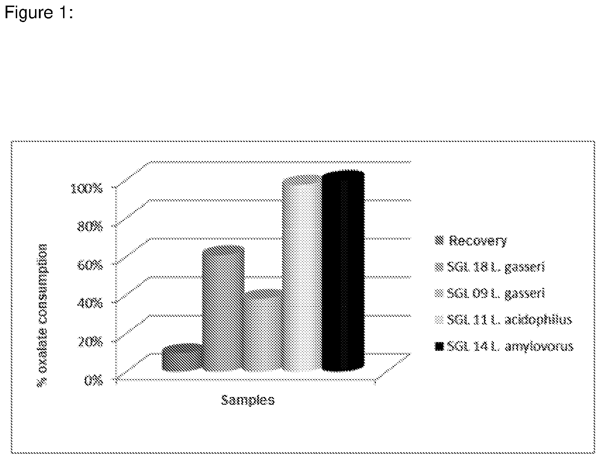

A method for measuring the concentration of oxalate in the culture media was developed using the Trinity Biotech diagnostic kit to assess the concentration of oxalate in urine. *Lactobacillus* strains were thus grown in the presence of 5 mM sodium oxalate and, after 5 days of incubation, the concentration of oxalate in culture media was measured. Since these strains were not able to develop in a medium having oxalate as the only source of carbon, MRS was the culture medium used. The results obtained from the oxalate concentration measurements in the culture media are shown in Table 6 and Figure 1.

**Table 6:**

| **Samples** | **OD 590 nm** | **mM oxalate** | **% consumption** |
|---|---|---|---|
| Recovery | 1.676 | 4.52 | 9.6 |
| SGL 18 *L. gasseri* | 0.66 | 1.78 | 60.63 |
| SGL 09 *L. gasseri* | 1.041 | 2.81 | 37.90 |
| SGL 11 *L. acidophilus* | 0.046 | 0.12 | 97.28 |
| SGL 14 *L. amylovorus* | 0.004 | 0.01 | 99.79 |

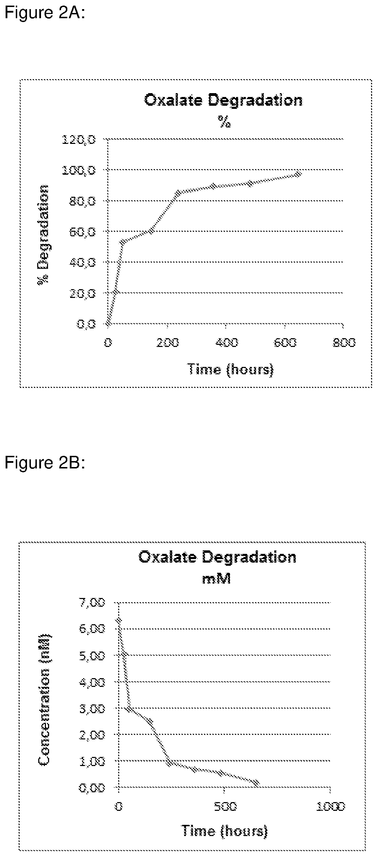

The kit used for this screening does not represent an accurate and precise method for the determination of oxalate. In the recovery tests, in fact, oxalate determinations were obtained with oxalate recovery values of 93%-107%, based on the amount added to the medium, thus demonstrating a rather high error of about 15%. This error, however, does not invalidate the degradation trend shown by the screening done on the *Lactobacillus* strains. The analyzed strains showed a different degree of metabolization of oxalate, and *L. amylovorus* SGL 14 was the most effective, showing a degradation of about 99.79% of oxalate added to the medium (see table). Since this strain is of great industrial interest, it was considered appropriate to choose it for subsequent studies and to characterize it using a HPLC technique in a curve describing oxalate consumption over time. The graphs obtained are shown in Figure 2A and 2B, showing both oxalate mM values consumed in the 27 days of monitoring, and the derived percentage values.

### Materials and Methods

### Assessment of oxalate degradation activity in probiotic strains in vitro

To assess the oxalate degradation activity in *L. amylovorus* SGL 14, the latter was grown for 5 days in MRS medium supplemented with 5 mM sodium oxalate. After development, the cells were collected and treated as follows, to remove any interfering substances with subsequent measurements:
addition of 2.7 mM EDTA, able to chelate ions
addition of 10 mM trichloroacetic acid, which precipitates proteins
sterilization and centrifugation, to remove viable bacterial cells
addition of activate carbon, to eliminate phenolic derivatives.

The concentration of oxalate in the supernatant was measured using Trinity Biotech kit for assessing the concentration of oxalate in urine. This kit uses the following reactions:

oxalate+O₂→2 CO₂+H₂O₂ (oxalate oxidase)

H₂O₂+MBTH+DMAB→Indamine+H₂O (peroxidase)

Oxalate is converted into carbon dioxide and hydrogen peroxide by oxalate oxidase, and by reacting hydrogen peroxide with 3-methyl-2-benzothiazolinone hydrazone (MBTH) and 3-(dimethylamino)benzoic acid (DMAB) in presence of peroxidase, it is possible to obtain a colored product, indamine, (λ=590 nm) whose absorbance value is directly proportional to oxalate concentration in the sample.

The samples were read in the spectrophotometer after an incubation time of 5 min required for color development. The reaction mixtures were consisting of:
- 1 mL reagent A (DMAB 3.2 mmol/L, MBTH 0.22 mmol/L, buffer pH 3.1)
- 50 µL of sample (treated as described above)
- 100 µL reagent B (oxalate oxidase 3000 U/L, per oxidase 100000 U/L)

For each experiment, a calibration curve was built with known standard oxalate concentrations, from which to extrapolate the concentration values of the unknown samples.

To validate this experimental procedure, a recovery test was carried out for each medium.

In order to verify the activity and build a consumption curve over time, the strain was cultured in the same growing conditions, and the medium analyzed at different times (0, 24, 48, 144, 240, 360, 480, 648 hours). Fermentation media are matrices very rich in various components, making it difficult to analyze and identify the active of interest if they are not previously treated. The preparation involved, after dilution in water, an elution step on SPE cartridge, and specifically a SPE STRATA C18-E 500mg/6mL is used (Phenomenex, Torrance, CA, USA). The same was activated with ethanol and water, and the sample and first wash eluate was collected for analysis. The eluate collected was evaporated under a slight nitrogen flow, taken up with water and injected into HPLC-DAD system (Agilent, Santa Clara CA, USA). This elution step is intended to remove all the "pollutants" fraction that would remain in the column, thus selecting only the fraction of interest. The oxalate chromatographic analysis was performed by HPLC/DAD with a specific column for organic acids, working with 100% monobasic potassium phosphate (KH₂PO₄) as a mobile phase, and detecting the analyte at 210 nm. The chromatographic analysis lasts a total of 9 min and the oxalate has a retention time of about 3.9 min.

### Example 8

### Sequencing of OXC and FRC genes in SGL 14

Sequencing of *oxc* (oxalyl-CoA decarboxylase) and *fcr* (Formyl CoA transferase) genes was performed by first amplifying the two genes using specific oxc-L/oxc-R and frc-L/frc-R primers obtaining two amplicons of the expected size: 1771 bp and 1363 bp, respectively. Sequencing was performed by Eurofins service and the sequences obtained were aligned with genes of other lactic bacteria already deposited. From the homology percentage between the *oxc* and *frc* genes of *L*. *amylovorus* SGL 14 and L. acidophilus LA 14 (98% and 85%, respectively); the presence of these genes in the *L. amylovorus* SGL 14 genome could be confirmed.

### Materials and Methods

### Sequencing of OXC and FRC genes in SGL 14

Genomic DNA was extracted from SGL 14 and analyzed by PCR for the identification of the oxc and frc genes already identified in other lactobacilli strains.

The components used in all amplification reactions are:
- Phusion high fidelity DNA polymerase (Thermo Scientific, Waltham, Massachusetts, USA) as thermostable DNA polymerase (2 U per reaction) and its reaction buffer 5x Phusion GC buffer;
- mixture of triphosphate nucleotides (8 mM, Euroclone, Siziano, Italy).
- primer set (100 µM, Eurofins MWG Operon, Ebersberg, Germany);
- genomic DNA;
- sterile water.

The following are the primers used, the thermocycle and the expected amplicon.

**Table 7:**

| Name | Gene | Sequence 5'-3' | Amplicon |
|---|---|---|---|
| oxc-L | Oxalyl Co A Decarboxylase | CTTGAAATGCAAGATGA-AAGCA SEQ ID NO:3 | 1771 bp |
| oxc-R | | CTTCAGTCA TT ATTT A TTCTCC SEQ ID NO:4 | |
| frc-L | Formyl CoA Transferase | GGAGAATAAATAATGACTGAAGA SEQ ID NO:5 | 1363 bp |
| frc-R | | CGGT AAAAATT AATT A TTCACC SEQ ID NO:6 | |

The amplicons obtained were sequenced and compared with *L. acidophilus* LA 14 genes already deposited in the database.

### Example 9

### Animal experimentation: efficacy assessment of SGL 14 and Phyllanto niruri association

Forty mice in good health were selected. Before the start of the trial, each animal was subjected to clinical examination including temperature and body weight recording.

The animals were then randomly divided into 4 groups:
1) negative control group fed with normal diet.
2) toxicity test group fed with normal diet and high-dose supplement prototype.
3) positive control group fed with high-ox diet.
4) efficacy test group fed with high-ox diet, with the addition of the high-dose supplement prototype.

The parameters monitored and the analyzes performed during the experimentation are summarized in Table 8 below.

**Table 8:**

| ***Parameters (Urine)*** | ***Parameters (Blood)*** |
|---|---|
| *Volume (ml*/*24h)* | *Haemochrom* |
| *pH* | *Lipid profile* |
| *Oxalate (µmol*/*24h)* | *Glucidic profile* |
| *Sodium (µmol*/*24h)* | *Plasma oxalate (µM)* |
| *Calcium (µmol*/*24h)* | *Urea (mg*/*dl)* |
| *Potassium (µmol*/*24h)* | *Creatinine (mg*/*dl)* |
| *Creatinine (µmol*/*24h)* | |
| *Creatinine Clearance (µl*/*min)* | |
| *Proteinuria (µg*/*24h)* | |
| *Albuminuria (µg*/*24h)* | |
| *Urine sediment analysis* | |
| ***Parameters (feces)*** | ***Organs removal*** |
| *Oxalate (µg*/*mg feci)* | *Cecum intestine (cecal oxalate)* |
| *Intestinal Microbiota characterization* | *Liver (anatomo-pathological changes)* |
| *L. amylovorus* search | *Kidney (anatomo-pathological changes)* |
| ***Anthropometric parameters*** | |
| *Body weight (g)* | |

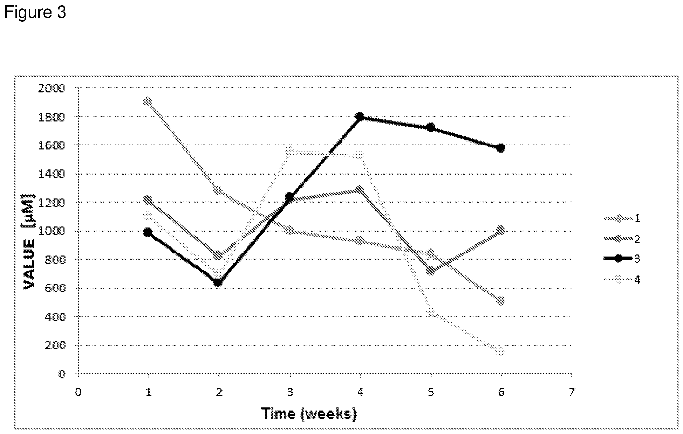

The results obtained from the measurements of oxalate concentration in the urine of mice in the 4 experimental groups are shown in Table 9 and Figure 3.

**Table 9:**

| **TIME** | **Overall comparison significance** | **Significant multiple comparison between pairs** |
|---|---|---|
| **1** | No | |
| **2** | **Yes** | **Group 1 vs Group 3** |
| **3** | No | |
| **4** | No | |
| **5** | **Yes** | **Group 1 vs Group 4** |
| **6** | **Yes** | **Group 1 vs Group 2;** |
| | | **Group 1 vs Group 4;** |
| | | **Group 2 vs Group 4;** |
| | | **Group 3 vs Group 4** |

At the end of the trial, *i.e.* at 6 weeks, there was a (statistically significant) decrease in urinary oxalate in mice treated with the supplement prototype consisting of SGL 14 in association with dry extract of *Phyllanto niruri.*

### Example 10

### Preparation of the compositions

Compositions according to the present invention were prepared, and all the associations surprisingly demonstrated probiotic activity and may be used both for medical use and for food use in beverages.

### 1. Composition comprising the bacterial strain L. amylovorus SGL 14

Bacterial strain *L. amylovorus* SGL 14. Examples of dosages are, for the ferment, from 1 billion/dose to 20 billion/dose, in the form of powders, capsules, tablets, granulates, drops and syrups, chewing gum.

### 2. Composition comprising the bacterial strain L. amylovorus SGL 14 and a plant extract of Phyllanto niruri

The bacterial strain *L. amylovorus* SGL 14 may be associated to the plant extract of *Phyllanto niruri* in a composition. Suitable dosages can be: SGL 14 from 1 billion/dose to 20 billion/dose, and from 0.5 g/dose to 10 g/dose for the extract of *Phyllanto niruri.* They will be formulated in powders, capsules, tablets, granulates, drops and syrups, chewing gum.

### 3. Composition comprising the bacterial strain L. amylovorus SGL 14 and the bacterial strain L. gasseri SGL 18

Suitable dosages for both ferments may be from 1 billion/dose to 20 billion/dose, in the form of powders, capsules, tablets, granulates, drops and syrups, chewing gum.

### 4. Composition comprising the bacterial strain L. amylovorus SGL 14, a plant extract of Phyllanto niruri and the bacterial strain L. gasseri SGL 18

Suitable dosages for SGL 14 and SGL 18 are in the range of 1 billion/dose to 20 billion/dose, and in the range of 0.5 g/dose to 10 g/dose for the extract of *Phyllanto niruri.* They will be in the form of powders, capsules, tablets, granulates, drops and syrups, chewing gum.

From the detailed description and from the Examples reported above, the advantages achieved by the bacterial strain *L. amylovorus* SGL 14 of the present invention are apparent. In particular, this bacterial strain has been shown to be surprisingly and advantageously suitable as a probiotic and in the treatment of calculi, hyperoxalurias and lithiasis.

## Claims

1. A bacterial strain belonging to the *Lactobacillus amylovorus* species, wherein said strain is *L. amylovorus* SGL 14 deposited with accession number DSM 27014 on March 18, 2013.

2. A composition comprising the bacterial strain according to claim 1.

3. The composition according to claim 2, further comprising a plant extract of *Phyllanto niruri.*

4. The composition according to claim 2, further comprising the bacterial strain *L. gasseri* SGL 18 deposited with accession number DSM 32802 on April 20, 2018.

5. The composition according to any one of claims 2 to 4, further comprising pharmacologically acceptable excipients.

6. The composition according to any one of claims 2 to 5, wherein said composition has probiotic activity.

7. The composition according to any one of claims 2 to 6, wherein said composition is in a solid or liquid form.

8. The composition according to any one of claims 2 to 7, for use as a medicament.

9. The composition for use according to claim 8, for use in treatment of calculi selected from the group consisting of kidney stones, gallstones and gallbladder stones.

10. The composition for use according to any one of claims 8 or 9, for use in treatment of hyperoxaluria.

11. The composition for use according to any one of claims 8 to 10, for use in treatment of lithiasis.

12. The composition according to any one of claims 2 to 7, which is a food composition.

## Patentansprüche

1. Bakterienstamm, der zur Spezies *Lactobacillus amylovorus* gehört, wobei es sich bei dem Stamm um L *amylovorus* SGL 14 handelt, der am 18. März 2013 unter der Zugangsnummer DSM 27014 hinterlegt wurde.

2. Zusammensetzung, umfassen den Bakterienstamm nach Anspruch 1.

3. Zusammensetzung nach Anspruch 2, ferner umfassend einen Pflanzenextrakt aus *Phyllanto niruri.*

4. Zusammensetzung nach Anspruch 2, ferner umfassend den Bakterienstamm L *gasseri* SGL 18, der am 20. April 2018 unter der Zugangsnummer DSM 32802 hinterlegt wurde.

5. Zusammensetzung nach einem der Ansprüche 2 bis 4, ferner umfassend pharmakologisch akzeptable Hilfsstoffe.

6. Zusammensetzung nach einem der Ansprüche 2 bis 5, wobei die Zusammensetzung eine probiotische Aktivität aufweist.

7. Zusammensetzung nach einem der Ansprüche 2 bis 6, wobei die Zusammensetzung in einer festen oder flüssigen Form vorliegt.

8. Zusammensetzung nach einem der Ansprüche 2 bis 7 zur Verwendung als Medikament.

9. Zusammensetzung zur Verwendung nach Anspruch 8, zur Verwendung bei der Behandlung von Steinen, ausgewählt aus der Gruppe bestehend aus Nierensteinen, Gallensteinen und Gallenblasensteinen.

10. Zusammensetzung zur Verwendung nach einem der Ansprüche 8 oder 9, zur Verwendung bei der Behandlung von Hyperoxalurie.

11. Zusammensetzung zur Verwendung nach einem der Ansprüche 8 bis 10, zur Verwendung bei der Behandlung von Lithiasis.

12. Zusammensetzung nach einem der Ansprüche 2 bis 7, die eine Lebensmittelzusammensetzung ist.

## Revendications

1. Souche bactérienne appartenant à l'espèce *Lactobacillus amylovorus* , dans laquelle ladite souche est L *amylovorus* SGL 14 déposée avec le numéro d'accession DSM 27014 le 18 mars 2013.

2. Composition comprenant la souche bactérienne selon la revendication 1.

3. Composition selon la revendication 2, comprenant en outre un extrait végétal de *Phyllanto niruri.*

4. Composition selon la revendication 2, comprenant en outre la souche bactérienne L *gasseri* SGL 18 déposée avec le numéro d'accession DSM 32802 le 20 avril 2018.

5. Composition selon l'une quelconque des revendications 2 à 4, comprenant en outre des excipients pharmacologiquement acceptables.

6. Composition selon l'une quelconque des revendications 2 à 5, où ladite composition présente une activité probiotique.

7. Composition selon l'une quelconque des revendications 2 à 6, où ladite composition se présente sous une forme solide ou liquide.

8. Composition selon l'une quelconque des revendications 2 à 7 à utiliser comme médicament.

9. Composition à utiliser selon la revendication 8, pour une utilisation dans le traitement des calculs choisis dans le groupe composé par les calculs rénaux, les calculs biliaires et les calculs de la vésicule biliaire.

10. Composition à utiliser selon l'une quelconque des revendications 8 ou 9, pour une utilisation dans le traitement de l'hyperoxalurie.

11. Composition à utiliser selon l'une quelconque des revendications 8 à 10, pour une utilisation dans le traitement de la lithiase.

12. Composition selon l'une quelconque des revendications 2 à 7, qui est une composition alimentaire.
